(19) Europäisches Patentamt
European Patent Office
Office européen des brevets

(11) Publication number: **0 147 540**
**B1**

(12) **EUROPEAN PATENT SPECIFICATION**

(45) Date of publication of patent specification: **11.07.90**

(51) Int. Cl.$^5$: **A 61 K 31/255**

(21) Application number: **84111578.5**

(22) Date of filing: **27.09.84**

(54) **Disulfamate for treating malignant conditions.**

(30) Priority: **29.09.83 US 537197**

(43) Date of publication of application:
**10.07.85 Bulletin 85/28**

(45) Publication of the grant of the patent:
**11.07.90 Bulletin 90/28**

(84) Designated Contracting States:
**BE CH DE FR GB IT LI NL SE**

(56) References cited:
**US-A-4 075 351**

(73) Proprietor: **STERLING DRUG INC.**
**90 Park Avenue**
**New York New York (US)**

(72) Inventor: **Bailey, Dennis Mahlon**
**4 Johnny Place**
**East Greenbush New York (US)**

(74) Representative: **Baillie, Iain Cameron et al**
**c/o Ladas & Parry Isartorplatz 5**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 147 540 B1

## Description

The present invention relates to a compound for use in treating malignant conditions in mammals and a method of such treatment by administration of such compounds, namely certain sulfamate compounds.

U.S. Patents 3,997,585 and 4,075,351 and Hirsch et al., J. Med. Chem. *24,* 901—903 (1981), describe a series of sulfamate compounds having male antifertility properties. The compounds have the formula

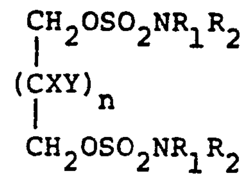

wherein n is an integer from 0 to 8 and X and Y are hydrogen, provided that when n is 1, X and Y are hydrogen, lower alkyl having 1—3 carbon atoms, phenyl, benzyl or phenethyl; $R_1$ and $R_2$ are hydrogen, alkyl having 1—7 carbon atoms, phenyl, benzyl, phenethyl or cycloalkyl having 5—6 carbon atoms. Compounds specifically disclosed include 1,6-bis-O-sulfamyl-1,6-hexanediol, 1,7-bis-O-sulfamyl-1,7-heptanediol, and 1,8-bis-O-sulfamyl-1,8-oxtanediol.

The invention resides in inhibiting the growth of malignant cells in a mammal by administering to said mammal an antineoplastically effective amount of a compound of the formula

$$H_2NSO_2O(CH_2)_nOSO_2NH_2$$

where n is 6—8, incorporated in a suitable pharmaceutically acceptable diluent or excipient.

Unexpectedly, it has been found that a series of alkanediol disulfamates, disclosed in the prior art as having male antifertility activity, possess antineoplastic activity as evidenced by their activity in reducing the size of tumors and increasing the survival time in murine species implanted with various tumors.

The antitumor activity lies essentially in homologs of the formula

$$H_2NSO_2O(CH_2)_nOSO_2NH_2$$

where n is 6—8, inclusive.

The alkanediol disulfamates were prepared according to the prior art method by reacting an alkanediol with sulfamoyl chloride in the presence of sodium hydride, as illustrated by the following procedure.

A 57% oil dispersion of sodium hydride (11.8 g, 0.28 mole NaH) was added to 150 ml of tetrahydrofuran. The suspension was stirred and gently heated, and 15.84 g (0.12 mole) of 1,7-heptanediol in 75 ml of tetrahydrofuran was added gradually over a period of 90 minutes. An additional 25 ml of tetrahydrofuran was then added, and the reaction mixture was stirred at reflux for five hours. An additional 150 ml of tetrahydrofuran was added, and to the stirred warm mixture there was added dropwise a solution of 28.9 g (0.25 mole) of sulfamoyl chloride in 150 ml of tetrahydrofuran over a two hour period. The reaction mixture was stirred at reflux for five hours, then cooled and treated dropwise with 125 ml of water. The mixture was acidified with 2N hydrochloric acid, and the organic layer was separated and dried over anhydrous sodium sulfate. The solvent was removed *in vacuo* and the residue triturated with ethyl acetate to give a yellow solid which was recrystallized from an ethyl acetate-hexane mixture to give 22.0 g of *1,7-heptanediol disulfamate* as colorless granules, m.p. 87—88°C.

By employing the same procedure but replacing the 1,7-heptanediol by a molar equivalent amount of 1,6-hexanediol by a molar equivalent amount of 1,6-hexanediol or 1,8-octanediol, there was obtained, respectively, *1,6-hexanediol disulfamate,* colorless granules, m.p. 122—124°C; and *1,8-octanediol disulfamate,* tan granules, m.p. 100—101°C.

The compounds described hereinabove were tested for antitumor activity in mice under the auspices of the National Cancer Institute (U.S. Dept. of Health and Human Services) following the test procedures and protocols set forth in *Cancer Chemotherapy Reports,* Part 3, Vol. 3, No. 2 (September 1972).

The compounds were tested against the following implanted tumors:

3B131 = B1—B16 melanocarcinoma
3LE31 = LE—L—1210 lymphoid leukemia
3MBG5 = MB—MX—1 mammary carcinoma xenograft
3PS31 = PS—P—388 lymphocytic leukemia
3CDJ2 = CD—CD8$F_1$ mammary adenocarcinoma

The results were determined at T/C (%) values, calculated in terms of mean survival rates (MST)

$$T/C\ (\%) = \frac{\text{MST (treated animals)}}{\text{MST (control animals)}} \times 100$$

or (for 3MBG5 and 3CDJ2) in terms of change in tumor weight according to the following procedures:

3MBG5: On day 0, tumor fragments with an average diameter of 9—12 ocular micrometer units (10 OMU = 1 mm; weight of fragment with a length and width of 10 OMU = 0.5 mg) are implanted beneath the renal capsule of athymic mice. On day 11, tumor measurements are taken again. All length (L) by width (W) measurements are converted to weight by the formula: wt. (mg) = (L × W × W)/2. For positive changes in test tumor weights (i.e. mean T.W. on day 11 — mean T.W. on day 0 was positive), T/C values are calculated from the test tumor weight change/control tumor weight change × 100. For negative changes in test tumor weights (tumors regressed), T/C values are calculated from the test tumor weight change/initial test tumor weight × 100.

3CDJ2: On Staging day and final evaluation day, the sizes of tumors of individual mice are measured according to the following formula: Wt (mg) = (L × W × W)/2. Change in tumor weight for each group is calculated. For positive changes in test tumor weights (i.e. median T.W. on final evaluation day — median T.W. on Staging day), T/C values are calculated from the test tumor weight change/control tumor weight change × 100. For negative changes in test tumor weights (tumors regressed), T/C values are calculated from the test tumor weight change/initial tumor weight × 100.

The tables below give the results obtained with the compounds pertinent to the instant invention. The compounds were tested as suspensions in polysorbate 80 (Tween® 80) and by intraperitoneal injection unless otherwise stated.

1,7-Heptanediol disulfamate

| Tumor Systems | Dose (mg/kg/inj) | T/C (%) MST | T/C (%) Weight Change |
|---|---|---|---|
| 3B131 | 50 | 171-181 | |
| 3LE31 | 50 | 141-171 | |
| 3MBG5 | 150 | | -97 |
| 3MBG5 | 75 | | -81 |
| 3PS31 | 100 | 199 | |
| 3PS31 | 50 | 174 | |
| 3CDJ2 | 250 | | -27, -36 |

II 1,6-Hexanediol disulfamate

| Tumor Systems | Dose (mg/kg/inj) | T/C (%) MST | Weight Change |
|---|---|---|---|
| 3B131 | 50 | 180 | |
| 3B131 | 25 | 168-185 | |
| 3LE31 | 25 | 158-178 | |
| 3MBG5 | 75[a] | | -24, -100 |
| 3PS31 | 100 | 294 | |
| | 50 | 193 | |

a) subcutaneous administration

III 1,8-Octanediol disulfamate

| Tumor Systems | Dose (mg/kg/inj) | T/C (%) MST | Weight Change |
|---|---|---|---|
| 3B131 | 25 | 132-140 | |
| 3LE31 | 50 | 186 | |
| 3LE31 | 25 | 146 | |
| 3MBG5 | 80[a] | | -87, -100 |
| 3PS31 | 50 | 174-179 | |
| 3CDJ2 | 250 | | -7 |
| 3CDJ2 | 125 | | 6 |

a) subcutaneous administration

For practice of the invention, the compounds are prepared for use by incorporating them in conventional, pharmaceutically acceptable, diluents, carriers or excipients. For parenteral administration (intravenous, intraperitoneal, subcutaneous or intramuscular), the compounds are dissolved or suspended in an aqueous or non-aqueous vehicle. For oral administration, the compounds are formulated in dosage unit form as tablets or capsules. Exemplary diluents, carriers or excipients include lactose, dextrose, sucrose, sorbitol, mannitol, starches, gum acacia, calcium phosphate, mineral oil, cocoa butter, alginates, tragacanth, gelatin, methyl cellulose, methyl- and propyl hydroxybenzoates, talc and magnesium stearate.

**Claims**

1. A compound for use inhibiting the growth of malignant cells in a mammal, said compound having the formula

$$H_2NSO_2O(CH_2)_nOSO_2NH_2$$

where n is 6—8.

2. A compound according to claim 1, wherein said compound is 1,7-heptanediol disulfamate.

3. A compound according to claim 1, wherein said compound is 1,6-hexanediol disulfamate.

4. A compound according to claim 1, wherein said compound is 1,8-octanediol disulfamate.

5. The use of a compound as defined in claim 1 for preparing a medicament for use in inhibiting the growth of malignant cells in a mammal.

**Patentansprüche**

1. Verbindung mit der Formel

$$H_2NSO_2O(CH_2)_nOSO_2NH_2$$

in der n den Wert 6 bis 8 hat, zur Verwendung zum Hemmen des Wachstums von bösartigen Zellen in einem Säuger.

2. Verbindung gemäß Anspruch 1, wobei die genannte Verbindung 1,7-Heptandiol-disulfaminsäureester ist.

3. Verbindung gemäß Anspruch 1, wobei die genannte Verbindung 1,6-Hexandiol-disulfaminsäureester ist.

4. Verbindung gemäß Anspruch 1, wobei die genannte Verbindung 1,8-Octandiol-disulfaminsäureester ist.

5. Verwendung einer wie in Anspruch 1 definiert Verbindung zur Herstellung eines Arzneimittels zur Verwendung als Wachstumshemmer für bösartige Zellen in einem Säuger.

**Revendications**

1. Composé servant à inhiber la croissance des cellules malignes chez les mammlfères, ledit composé répondant à la formule

$$H_2NSO_2O(CH_2)_nOSO_2NH_2$$

dans laquelle n est compris entre 6 et 8.

2. Composé selon la revendication 1, dans lequel ledit composé est le disulfamate de 1,7-heptandiol.

3. Composé selon la revendication 1, dans lequel ledit composé est le sulfamate de 1,6-hexanediol.

4. Composé selon la revendication 1, dans lequel ledit composé est le sulfamate de 1,8-octanediol.

5. Utilisation d'un composé selon la revendication 1, pour préparer un médicament servant à inhiber la croissance de cellules malignes chez les mammlfères.